Europäisches Patentamt

European Patent Office

Office européen des brevets

(19)

(11) Publication number: **0 250 261**
**A1**

(12) # EUROPEAN PATENT APPLICATION

(21) Application number: **87305471.2**

(22) Date of filing: **19.06.87**

(51) Int. Cl.⁴: **C 12 N 15/00**, C 12 N 7/00,
A 61 K 39/015, C 12 P 21/00

(30) Priority: **20.06.86 GB 8615068**

(43) Date of publication of application: **23.12.87**
**Bulletin 87/52**

(84) Designated Contracting States: **AT BE CH DE FR GB IT LI LU NL SE**

(71) Applicant: **THE WELLCOME FOUNDATION LIMITED,
183-193 Euston Road, London NW1 2BP (GB)**

(72) Inventor: **Holder, Anthony Arthur, The Wellcome
Research Laboratories Langley Court, Beckenham Kent
(GB)**
Inventor: **Lockyer, Michael James, The Wellcome
Research Laboratories Langley Court, Beckenham Kent
(GB)**

(74) Representative: **Stott, Michael John et al, The Wellcome
Research Laboratories Group Patents & Agreements
Langley Court, Beckenham Kent BR3 3BS (GB)**

(54) Vaccines containing epitopes of both the CSP and a blood stage antigen of a plasmodium parasite.

(57) There is provided conjugate, recombinant protein comprising at least one epitope of each of the CSP and a blood stage antigen of a Plasmodium parasite. The protein produced is effective in raising an immune response to both the sporozoite and merozoite stage of the Plasmodium life cycle.

0250261

Novel Vaccines

The present invention relates to a protein capable of inducing immunity to malaria when administered as a vaccine.

Malaria is an increasing health problem throughout the world. Several hundred million people suffer from the disease and the most acute form, caused by the protozoan parasite Plasmodium falciparum, kills over a million children each year in Africa alone.

The life cycle of the parasitic protozoan Plasmodium falciparum is complex, requiring both the mosquito and the mammalian host, man for completion. The infection of man is initiated by the inoculation of sporozoites in the saliva of an infected mosquito. The sporozoites migrate to the liver and there infect hepatocytes where they differentiate, via the exoerythrocytic intracellular stage, into the merozoite stage which infects the blood to initiate cyclical replication in the asexual blood stage. The cycle is completed by the differentiation in the blood of sexual stage gametocytes which are ingested by the mosquito, where they develop through a series of stages in the midgut to produce sporozoites which migrate to the salivary gland.

The mechanism of malarial immunity is complex, but is thought to involve both antibody and cell-mediated components. At the sporozoite stage the surface of the organism appears to be covered with a single protein, the circumsporozoite protein (CSP), containing an immunodominant repeating epitope. The role of antibody in mediating protection against this form is confirmed by the fact that antibodies against the repeat will abolish infectivity and block the invasion of hepatocytes (Hollingdale et al., J. Immunol. 132, (1984) 909; Mazier et al., Science 231, (1985) 156). The structure of this repeat has been shown to be constant in several strains of P. falciparum and the repeat sequence has been produced by both synthetic and recombinant methods. The asexual stage is more complex and the parasite produces many proteins that interact with the immune system. One protein of high molecular mass (precursor to the major antigens on the merozoite surface) has been shown to induce immunity to the asexual stage in

MJWD/AS/12th June 1987

animal models. This class of antigens is present in all species of malaria studied so far and, in P. falciparum, has a molecular mass of about 195,000 (as determined by polyacrylamide gel electrophoresis). This protein is referred to herein as P.195.

The circumsporozoite protein gene of P. falciparum has been cloned and sequenced by two groups (Dame et al., Science 225, (1984) 593; Enea et al., Science 225, (1984) 625). The gene contains a central region of the tandemly repeated tetrapeptide Asn-Ala-Asn-Pro (repeat A) repeated 37 times and interspersed with 4 minor repeats Asn-Val-Asp-Pro (repeat B) in a Brazilian P. falciparum isolate. It is probable that this repetitive sequence is a strong immunogen and capable of inducing anti-sporozoite immunity. In the U.S.A., Phase I clinical trials of a synthetic anti-sporozoite vaccine are underway. The vaccine comprises either A) a recombinant protein containing 32 immunodominant tetrapeptide repeats from the central repeat region of the gene or, B) a synthetic peptide consisting of three tetrapeptide repeats.

In vitro data show that the smallest number of synthetic or bacterially- expressed tetrapeptides needed to inhibit binding of monoclonal antibodies to extracts of sporozoites is 3 (Ballou et al., Science 228, (1985) 998; Zavala et al., Science 228, (1985) 1436; Young et al., Science 228, (1985) 958). The latter group expressed the CSP as N-terminal fusion proteins of 16, 32 and 48 tandem copies with 32 amino acids of an out-of-frame Tet$^r$ gene. The basic unit for the bacterial constructs was a fragment of the CSP gene coding for 16 repeats. Purified proteins containing 32 or 48 repeats induced high antibody titres in mice without adjuvants, whereas proteins with 16 repeats required alum or Freund's complete adjuvant (FCA) for antigenicity. Antibodies against recombinant proteins reacted with CSP on live sporozoites and almost totally blocked invasion of human hepatoma cells in vitro. These findings suggest that use of a recombinant anti-sporozoite vaccine may be effective since antibody titres correlate well with immunity to sporozoite infection (Nardin et al., J. Exp. Med. 156,(1982) 20), and all isolates of P. falciparum sporozoites tested to date contain the immunodominant Asn-Ala-Asn-Pro repeat (Weber and Hockmeyer, Mol. Biochem. Parasitol. 15, (1985) 305; Zavala et al., J. Immunol. 135, (1985) 2790).

MJWD/AS/12th June 1987

P. 195, which appears to be a glycoprotein (Howard, R.J. et al., Mol. Biochem. Parasitol. 11, (1984) 349) is synthesised within the schizont form of the parasite and, at the end of the intra-erythrocytic stage the antigen is proteolytically processed into discrete fragments (Holder and Freeman, J. Exp. Med. 156, (1982) 1528; Hall et al., Mol. Biochem, Parasitol. 11, (1984) 61. On the surface of the merozoites (released into the plasma at the end of the intra-erythrocytic stage), the protein is fully processed, with three discrete fragments of P. 195 having molecular weights of about 83,000, 42,000 and 19,000 being present and recognised by polyclonal anti P.195 antibodies. These three fragments are major surface antigens of merozoites and are strongly recognised by human immune serum (Freeman and Holder, J. Exp. Med. 158, (1983) 1647, Holder and Freeman, J. Exp. Med. 160, (1984) 624).

Cloning and immunological data for P.195 are described in European Patent Application No. 85301173.2.

One problem with a sporozoite vaccine alone is that since this and the blood stage are antigenically distinct, failure to neutralise 100% of sporozoites would still permit infection of the liver and eventually lead to a full scale malarial infection. Likewise, a vaccine directed solely against the blood stage would have to contend with a malarial infection arising full-blown from the liver.

It has now been discovered that a recombinant, conjugate protein, comprising fragments of both CSP and blood stage antigens, is capable of inducing immunity against both the sporozoite and blood stages of the Plasmodium life- cycle.

Thus, the invention provides, in a first feature, a recombinant, conjugate protein comprising at least one epitope of each of the CSP and a blood-stage antigen of a Plasmodium parasite.

The term 'epitope' as used herein denotes an immunogenic determinant of an immunogenic molecule, the immunogenic determinant comprising a molecular configuration capable of eliciting a protective immune response in a susceptible animal, when presented in a suitable form.

MJWD/AS/12th June 1987

There are several advantages associated with a vaccine containing such a protein. In the first instance, even should the immune response raised against the sporozoite stage fail, the resulting, initial blood infection will be greatly diminished, permitting greater control and suppression of the parasite by antibodies against this stage. This secondary response might otherwise be swamped by sheer numbers of invading parasite. Since each sporozoite that successfully invades a liver cell gives rise to several tens of thousands of merozoites when the liver schizont bursts, even a modest reduction in sporozoite viability will have a dramatic effect on the initial blood stage infection.

Secondly, vaccination pressure could lead to antigenic variation on the parasite, rendering a monovalent vaccine useless. The probability associated with such an event occurring twice, to render a bivalent vaccine ineffective, are infinitessimally small.

Further advantages of a vaccine containing a protein according to the invention arise from: purification - a bivalent vaccine comprising two separate proteins would require two separate purifications, doubling the chances of retaining an undesirable impurity; production - only one organism need be cultivated to synthesise the one protein, instead of two, requiring double the facilities, and; benefits to the recipient - additional (often bacterial) protein sequence is an undesirable, but usually unavoidable part of any recombinant protein, and should be kept to a minimum. When only one protein is necessary, the presence of additional sequence is reduced.

In a further aspect, the present invention provides a recombinant, conjugate protein as described above, wherein the Plasmodium species is P. falciparum.

P.falciparum is the commonest cause of acute malaria and is the species against which travellers require protection.

MJWD/AS/12th June 1987

The present invention also provides a recombinant, conjugate protein as described above, wherein the blood stage antigen component is P.195.

As described above, tests have shown that P.195 is capable of inducing immunity to malaria when administered as a vaccine. In a further aspect of the present invention, there is provided a recombinant, conjugate protein as described above, wherein the blood stage antigen component is a naturally occurring fragment of P.195, especially the 42kD fragment and particularly the C terminal end of the 42kD fragment.

As described in European Patent Application No. 8504429, P.195 is cleaved in vivo into various fragments of sizes of about 83kD, 42kD and 19kD. The 42kD fragment in particular seems to be associated with a protective immune response, especially sequences derived from its C-terminus.

The invention further provides a recombinant, conjugate protein as described above wherein the shortest sequence containing a CSP epitope is selected from the group, A(n+1) A(n)B,BAB,ABA, BA(n) wherein A is Asn-Ala-Asn-Pro, B is Asn-Val-Asp-Pro, and n is an integer $\geqslant 2$.

Advantageously, the proteins described above are presented in the form of a vaccine. Thus, the present invention provides, in an alternative embodiment, a protein as described above in the form of a vaccine.

It will be appreciated that invention also lies in the DNA sequence required to code for any protein described above.

Thus, the present invention provides a DNA sequence encoding a protein as described above.

The DNA sequence according to the invention may correspond to naturally occurring sequences encoding the desired antigens, or it may correspond to a mutated form of such sequences, possible mutations including single or multiple base substitutions, deletions, insertions and inversions, but with the sequences, in any event, always encoding,

MJWD/AS/12th June 1987

in either positive or negative sense, at least one epitope of each of the CSP and a blood-stage antigen.

The DNA sequence according to the invention may encode a peptide of greater or lesser length than any desired peptide fragment as DNA manipulation techniques do not necessarily conveniently give rise to DNA sequences corresponding to desired peptides. Thus, for example, a DNA sequence substantially encoding a fragment of P.195 may not encode the first 30 amino acids of the fragment or, alternatively, it may also encode the last 30 amino acids of the preceding fragment (where relevant).

In a yet further aspect of the invention there is provided a non-pathogenic virus provided with a DNA sequence according to the invention. This may be used to provide immunity to malaria in a susceptible vertebrate host.

Such immunity arises after infection of the host by the virus, whereby the virus replicates, producing the proteins for which it codes including the protein according to the invention. Release of virions from the cell also allows release of the immune protein, thus giving rise to immunity efficiently and requiring no costly pre-synthesis of protein, but mere breeding of virus.

The invention also provides a non-pathogenic virus as defined above, further capable of providing immunity to other infection(s) which may be administered jointly, or individually, together with any other vaccine.

Another feature of the invention provides an expression vector, containing a DNA sequence according to the invention, tandemly linked to an amino- terminal coding portion of a gene translatable by a host and, optionally, a control sequence(s) associated therewith.

In a further feature of the present invention there is provided a fusion protein comprising a host peptide, and a recombinant, conjugate protein as described above.

MJWD/AS/12th June 1987

In an alternative aspect of the invention, there is provided an expression vector comprising a gene capable of translation in a suitable host, optionally provided with relevant control sequences, into which is inserted a DNA sequence according to the invention suitably altered such that the portion of said gene coding for the host peptide, together with the inserted DNA are correctly translated on expression in a suitable host to produce a fusion protein as described above.

Thus, the invention further provides a vector containing a DNA sequence as described above. It further provides an expression vector containing a DNA sequence as described above which is tandemly linked to a coding portion of a gene translatable by a host and optionally a control sequence(s) associated therewith.

According to a further feature of the present invention there is provided a method which comprises transforming a host cell with a cloning vector comprising the DNA sequence according to the invention and culturing the host cell to provide expression of a protein as described above.

According to a further feature of the present invention there is provided a vector containing the DNA sequence according to the present invention.

In a further aspect of the invention, there is provided a vector containing the DNA sequence according to the invention, further containing one or more control sequences to regulate the expression of said DNA sequence.

In a further embodiment of the invention there is provided a method for synthesising a protein as described above, said protein being optionally covalently linked to a further peptide sequence, which method comprises the steps of:

a) creating a cDNA or genomic DNA library from Plasmodium falciparum;

b)   selecting probes for CSP and blood-stage antigen DNA and
     rendering the said probes radio-active;

c)   ··selecting a member or members of the said library by use of
     the said probes; and

d)   using DNA thus selected from the said library to transform a
     suitable host which may be used to express the said protein.

There is in a further aspect provided a method for inducing immunity
to malaria in a susceptible vertebrate host, comprising the
administration of an effective amount of a vaccine, as herein before
defined, to the host.

Viruses may be used as hosts for the DNA sequence according to the
invention. For example, a strain of vaccinia virus (Tk$^-$) unable to
confer upon infected cells the ability to grow on media not containing
hypoxanthine is used to infect a tissue culture.  The tissue culture
may then be transformed with a DNA sequence as described above linked
to a Tk$^+$ genetic determinant.  Some of the subsequent viral progeny
will have such transforming sequence in the form of inserts in their
genomes.  These can then be selected by their ability to confer upon
tissue culture cells the ability to grow on media devoid of
hypoxanthine. Alternatively, if a tk$^+$ vaccinia virus growing in a cell
transfected with a tk$^-$ DNA recombines to acquire the tk$^-$ phenotype
(and the DNA sequence) then these recombinants will be resistant to
5-bromo deoxyuridine.  Such cells as do grow, are then further
selected for production of malarial antigen for example by use of
human immune serum..  Such vaccinia strains can then be used to
immunise mammals (including man) susceptible to malaria as the new
vaccinia strain will cause production of an immunogenic malarial
protein.

It will be appreciated that such vaccines are also readily capable ·of
providing immunity to other infections, such as smallpox, diphtheria,
hepatitis B, rabies, herpes simplex virus, whooping cough, HIV and the
like, by incorporation of relevant immunogens.

The insertion of a piece of foreign DNA into an E.coli gene in the
correct reading frame allows the expression of a fusion protein, in which part of the amino acid sequence is derived from the E.coli gene and part of it is derived from the inserted DNA. Suitable expression vectors with appropriate control sequences and convenient restriction sites have been constructed, which allow high levels of expression of fusion proteins.

Thus, examination of the restriction map of the expression system of choice and the sequences to be expressed, together with a knowledge of the translational frame, enable specific DNA fragments to be ligated into the expression vector and expressed, without further manipulation. For example, pWRL507 is a plasmid constructed from pAT153 and the trpE gene (Nichols et al., J. Mol. biol. 146, (1981) 45) with a synthetic EcoRI-Bgl II linker inserted at the Bgl II site at nucleotide 1223 in the trpE gene.

By using suitable restriction enzyme sites, a DNA sequence as described above may be cloned into a site within the trpE gene in the correct orientation, but usually in the wrong translational frame. In order to obtain expression of the inserted sequence, a synthetic linker of suitable length can be inserted into the restriction site between the trpE gene and the insert, to give in-frame expression of the fusion protein. Alternatively the plasmid containing the inserted DNA can be opened at the unique restriction site between the bacterial and the inserted DNA and the DNA treated briefly with the enzyme Bal 31 to remove a few bases from each end of the linearised DNA. After repair with the large (Klenow) fragment of DNA polymerase 1, the plasmid is recircularised with T4 ligase and used to transform bacteria. One in three of the transformants should contain the DNA sequence in the correct reading frame to be expressed as a fusion protein with the trpE gene product. It will be appreciated by those skilled in the art that the extent of digestion with Bal 31 will determine the final size of the expressed fusion protein and the relative lengths of trpE and the inserted sequence contained within it. In addition, the insertion of a synthetic linker during ligation after Bal 31 digestion and repair facilitates the analysis of particular strains after transformation. By the judicious use of specific restriction enzyme digestion and Bal 31 enzyme treatment, any

MJWD/AS/12th June 1987

specific region of a malarial protein antigen can be expressed as a fusion protein.

Alternatively, nuclease S1, which preferentially degrades single-stranded DNA, can be used to digest away the 'sticky' end of the Bgl II restriction site to leave a 'blunt' end. Recircularisation of the plasmid will then put the inserted DNA in a new, and possibly correct, reading frame.

An alternative method for expressing DNA fragments is to use an open reading frame (ORF) vector into which (usually) short pieces of DNA can be inserted, often within the sequence coding for the N-terminal amino acid sequence of an E.coli protein. The inserted DNA must contain no stop codons in the correct translational frame, be in the correct orientation relative to the direction of transcription, and be in the correct frame at each end. For pieces of DNA from a protein coding sequence generated by a random cleavage method the theoretical probability of read-through in the correct frame is 1 in 18. ORF vectors based on β-galactosidase have been described (Koenen et al., EMBO.J. 1, (1982) 509). Insertion of a piece of DNA into a site at the N-terminus of the protein in the correct frame confers read-through expression of the β-galactosidase protein which can be detected by hydrolysis of the chromogenic substrate 5-bromo-4-chloro-3-indolyl-β-D-galactoside (Xgal). For example if Xgal is included in the agar upon which the colonies are grown, transformation of a suitable host strain with a plasmid expressing a functional β-galactosidase will produce a blue colony. One such vector, pXY460, contains the β- galactosidase gene under the control of the tac promoter. Insertion of DNA into the Sma I site next to the EcoRI site may convert the gene to in-frame expression. Transformation of an E.coli host such as JM105 converts the bacteria to ampicillin resistance and expression of the fusion protein can be induced at high levels by the addition of isopropyl-β-D-thiogalactopyranoside (IPTG).

The host-encoded sequence in a fusion protein may be cleaved from that fusion protein by enzymic or chemical cleavage of the appropriate peptide bond. It will be apparent to those skilled in the art which

MJWD/AS/12th June 1987

enzymic or chemical cleavage method should be employed, by examination of the amino acid sequence expressed. By insertion of a synthetic oligonucleotide linker between the malarial DNA sequence and the bacterial gene sequence in the fusion protein expression system, a suitable site may be provided for enzymic or chemical cleavage between the malarial sequence and the remainder of the expressed fusion protein. In this way the malarial protein fragments may be purified away from host peptides.

Direct expression of the coding sequence for the above-described protein can be achieved by placing the inserted DNA sequence directly after an AUG start codon in the correct reading frame such that the DNA insert replaces the coding sequence normally transcribed and translated by the bacterial control region. Such a control region includes a promoter and a ribosome binding site in the optimal position relative to the start codon. The DNA sequence to be expressed may be correctly positioned by the use of suitable restriction sites and if necessary by using a suitable synthetic oligonucleotide linker. At the end of the insert DNA sequence a stop codon may be inserted in the correct reading frame to stop translation, and a terminator sequence to stop transcription may be added. The inserted DNA to be expressed may be the entire coding sequences of CSP and blood-stage antigen or the entire sequence from which the amino-terminal signal sequence has been removed, or preferably parts of the coding sequences corresponding to immunogenic fragments of the proteins. Suitable fragments may be prepared by restriction enzyme digestion of a suitable DNA clone (after examination of the nucleotide sequence), and if necessary further treatment at either or both ends with Bal 31 to digest away in a controlled way parts of the DNA sequence. Controlled digestion is achieved by the selection of proper buffer, temperature, reaction time and amount of enzyme. At this stage a suitable synthetic linker may be added, preferably by blunt end ligation to the insert, to provide an AUG start codon or facilitate ligation into the expression vector.

Controlled expression of any cloned fragment will be possible by use of the sequences at either end of the sequence, or by use of other sequences already known. Such sequences include promoters and

MJWD/AS/12th June 1987

enhancers.  Examples of such promoters include lac, trp, bacteriophage λ
pL and hybrid trp-lac(tac).  Suitable enhancers include the SV40
enhancer and the enhancer from bovine papillomavirus.

The vector referred to above may be any appropriate vector which is
suitable for the cloning of the DNA and which may be used to transform
a host cell and thereby express the relevant protein.  Such vectors
include plasmids, bacteriophages and cosmids.  Vectors which may be
used for cloning cDNA include pUC8, pUC9, pAT153, pBR325 and PBR328
for use in Escherichia coli, pBD9 and pKT438 for use in Bacillus
subtilis, pMA56 for use in yeast and pAdD26SV(A)-3,pSV2-dhfr,SVEHA3
and SVLHA8 for use in mammalian cells.  Other vectors may be used in
other expression systems, for example, vaccinia virus and baculovirus
in an insect cell system.

Vectors for use in expression of the relevant protein will include
control sequences, such as mentioned above.  Such vectors include
pXY460 and pWRL 507 for use in E.coli or pSV2-dhfr for use in
mammalian cells.

Examples of suitable host cells for use in the above-described method
may be prokaryotic, such as bacteria (for example E.coli HB101 and
DH1, B. subtilis sp.BD170 and IH6140), or eukaryotic, such as yeast
(for example XV610-8C yeast cells), insect or mammalian cells (for
example simian CV-1 cells).

Vaccines according to the invention conveniently comprise the said
protein together with a pharmaceutically acceptable carrier.
Pharmaceutically acceptable carriers, in this instance, are liquid
media suitable for use as vehicles to introduce the antigen into the
patient.  An example of such a carrier is saline solution.  The
protein according to the invention may be in solution or suspended as
a solid in the carrier, or it may be solubilised by the addition of
pharmaceutically acceptable detergent.

The vaccine may also comprise an adjuvant for stimulating the immune
response and thereby enhancing the effect of the vaccine.  A

MJWD/AS/12th June 1987

convenient adjuvant for use in the present invention is aluminium hydroxide.

Conveniently the vaccines are formulated to contain a final concentration of protein in the range of from 0.2 to 5 mg/ml, preferably 0.5 to 2 mg/ml, most preferably 1 mg/ml. After formulation the vaccine may be incorporated into a sterile container which is then sealed and stored at a low temperature, for example $4^{o}C$, or it may be freeze-dried.

In order to induce immunity in vertebrate hosts to malaria one or more doses of the vaccine suitably formulated may be administered. It is recommended that each dose is 0.1 to 2ml preferably 0.2 to 1ml, most preferably 0.5ml of vaccine.

The vaccines may be administered by any conventional method for the administration of vaccines including oral and parenteral (eg. subcutaneous or intramuscular) injection. The treatment may consist of a single dose of vaccine or a plurality of doses over a period of time.

The following examples are for illustration only and are not intended to limit the invention in any way.

In order to ease understanding of the following Examples the following Figures, relating to Examples as shown in the Table below are provided:

| Figure | Example |
|--------|---------|
| 1 | 2 and 7(c) |
| 2 | 2 |
| 3 | 7(a) |
| 4 | 7(c) and 8 |
| 5 | 8(a) |
| 6 | 8(b) |
| 7 | 9 |
| 8 | 9 |

-13a-

Example 1:    Cloning and Sequencing the CSP Gene

The CSP gene of P. falciparum was cloned and sequenced as follows.
Asynthetic 21-mer oligonucleotide,
5'TGCATTTGGGTTTGCATTTGG3', corresponding to the non-coding strand of
the Asn-Ala-Asn-Pro repeat was used as a probe to isolate the CSP gene
from P. falciparum.  Genomic DNA was digested with mung bean nuclease
in the presence of formamide to generate gene-size fragments (Dame et
al., supra) and Southern blotting experiments using the probe showed a
major hybridising fragment of 1.7kb.  Digested DNA in this size range
was ligated into the vector pUC9.  About 5000 recombinants were
obtained and 8 of these gave a strong positive signal when screened
with the $^{32}$P-labelled oligonucleotide probe.  Most of these positive
recombinants contained an insert of 1.7kb and one, pCSP6, was investigated further. Partial restriction mapping and sequencing of this clone confirmed that it contained the CSP gene. The region contained a total of 43 (37) A repeats and 3 (4) B repeats (the figures in brackets are nos. in the published Brazilian Strain). The repeats were organised as $ABABA_{15}BA_{26}$ in our strain and $ABABABA_{15}BA_{19}$ in the Brazilian clone (Lockyer and Schwarz, Mol. Biochem. Parasitol. 22: (1987) 101).

Example 2:    Expression of part of the CSP Gene as a hybrid with betagalactosidase

Fokl/Tthlll 1 digestion of the gene gave a 583bp fragment containing all the repeat region and a short length of flanking sequence. This fragment was treated with BAL-31 to randomize the ends, polished with Klenow and ligated into the SmaI site of the open reading frame vector pXY460. Constructs were obtained expressing the repeats as N-terminal fusions with $\beta$-galactosidase. Induction of one of these recombinants, pCSP 600/3, with IPTG produced a fusion protein with an apparent molecular mass of about 140,000 and detected by Coomassie blue staining of cell lysates after SDS polyacrylamide gel electrophoresis. This protein also reacted on a Western blot with a monoclonal antibody specific for the CSP, and antibodies raised to the synthetic peptide-carrier conjugate described in Example 10. The sequences of the junctions of the insert with plasmid vector were determined by plasmid-primed sequencing (Chen and Seeburg, DNA 4: (1985) 165). In pCSP 600/3 the insert is nucleotide 428 to 1011.

Example 3:    Cloning, Sequencing and Expression of the P.195 Gene

This work has been described in detail in Patent Application EP 8504429 Al and Nature, 317, (1985) 270 and Parasitology 94, (1987) 199. Several additional fragments derived from P.195 were detected on the merozoite and it was found that the 19kd species represents a subfragment derived from C-terminus of the 42kd species. Additional fragments of 38, 30 and 28kd were observed and localized approximately within the linear gene sequence.

MJWD/AS/12th June 1987

Example 4:  Effect of Antibodies against P.195 in an In Vitro Inhibition Assay: Identification of a region of the protein capable of inducing invasion inhibiting antibodies.

Parts of the P195 gene were placed in an expression vector to produce either β-galactosidase or anthranilate synthase fusion proteins (Holder et al Parasitology (1987) supre). The fusion proteins were used to raise antibodies in rabbits. An immunoglobulin fraction was prepared from the sera and added at a final concentration of about 2mg/ml to in vitro cultures of Plasmodium falciparum. The parasite was cultured in RPMI 1640 supplemented with 8% human serum and human erythrocytes at an haematocrit of 2% and a starting parasitaemia of 0.5% schizonts. The ability of the IgG fraction to inhibit merozoite invasion was measured by counting the number of parasitised red cells in Giemsa stained smears after a period of 24 hours and comparing this number with that in a control culture to which no rabbit antibody or antibody from a non-immunised animal had been added.

Only fusion proteins that contained the last (C-terminal) 420 amino acids of the P.195 sequence induced antibodies that would inhibit invasion by up to 50%. One fusion protein containing the last 100 amino acids before the hydrophobic tail sequence, induced antibodies that were almost as effective as those containing the entire terminal 420 amino acids, Table 1.

MJWD/AS/12th June 1987

Table 1:   Inhibition of in vitro invasion by antisera raised to
            recombinant protein

| Plasmid producing fusion protein | P.195 nucleotide sequence | % Inhibition over 24 hrs. |
|---|---|---|
| pME1 (507 pPfg1 Ndel-EcoRI) | 876-3559 | <5 |
| pME2 (507 pPfc 1028 EcoRI-Hind III) | 3555-5920 | 25 |
| pME3 (507 pPfc 1028 EСoRI-Nde 1) | 3555-4759 | <5 |
| pME11 (507/460 pPfc1028 Bam H1-Pst 1/4) | 4043-5260 | 30 |
| pME13 (460 pPfc 1028 Dde 3-10) | 4926-5230 | 35 |
| pME14 (460 pPfc 1028 Dde 2-9) | 3120-4910(approx) | <5 |
| pME15 (507 1028 EH Bal 31-3) | ~4000-5920 | 50 |
| pME16 (507 1028 EH Bal 31-9) | ~4000-5920 | 20 |
| pME17 (507 pPf 1028 EcoRI-Bam H1) | 3559-4028 | 15 |

Example 5:      Vaccination of Aotus monkeys with recombinant proteins
                and merozoites - protection against challenge infection
                with hybrid proteins containing P195 sequences.

a)    Material used for immunisation.

Merozoites were prepared from in vitro cultures of P. falciparum
(Freeman & Holder, J. Exp. Med. 158 (1983) 1647). The fusion protein
products of two E. coli clones, 507/460 pPfc 1028 BamH1-Pst1/4 (also
renamed as pME11) and 460 pPfc 1028 Dde 3-10 (also renamed as pME13)
were partially purified.  In 507/460 pPfc 1028 Bam H1-Pst1/4 the
fusion protein is a hybrid with the trpE gene product and contains
amino acids expressed from nucleotides 4043 to 5260 in the P.195 gene
sequence (Holder et al., Nature 1985).  In 460 1028 Dde 3-10 the
fusion protein is a hybrid with β-galactosidase and contains amino
acids expressed from nucleotides 4926 to 5230.  The trpE fusion was
purified from cell lysates on the basis of its insolubility in a
number of extraction buffers.  The galactosidase hybrid was purified
by affinity chromatography (Steers, Cuatrecases & Pollard, J. Biol.
Chem. 246 (1971) 196).

MJWD/AS/12th June 1987

b)    Immunisation protocol.

Three groups of animals were used:

Group 1:    Negative control.  Mixed 0.5ml 0.85% saline with 0.5ml
            Freund's Adjuvant.  Injected 0.2ml/animal (3 animals).

Group 2:    Positive control - immunization with whole parasites.
            Two vials each containing $4 \times 10^9$ merozoites in 0.2ml
            were made up to 0.5ml with saline and mixed with 0.5ml
            Freund's    Adjuvant.        Injected    0.2ml    ($10^9$
            merozoites)/animal (3 animals).

Group 3:    Test group - immunization with test antigen produced by
            recombinant   DNA   methods.   A   solution   of   0.6ml
            containing approximately 1.2mg of each fusion protein
            in saline was mixed with 0.6ml Freund's Adjuvant.
            Injected 0.2ml/animal (4 animals).

The three groups of animals were immunised according to the
schedule outlined below:

Day 0:      Primary immunization with antigens in Freund's Complete
            Adjuvant, intramuscular.  Fifty microlitre sample of
            blood taken from each animal to measure preimmunization
            antibody titre.

Day 14:     Fifty microlitre sample of blood taken from each animal
            to measure antibody titre.

Day 28:     Secondary immunization with antigens in Freund's
            Incomplete Adjuvant, intramuscular.

Day 42:     Tertiary immunization with antigens in Freund's
            Incomplete Adjuvant, intramuscular.  Fifty microlitre
            sample of blood taken from each animal to measure
            antibody titre.

MJWD/AS/12th June 1987

Day 48:   Infected donor monkey with <u>Plasmodium falciparum</u>.

Day 53:   Removed 1ml blood sample from each monkey (except donor) for testing.

Day 62:   Challenge infection by intravenous injection of <u>Plasmodium falciparum</u>-infected Aotus erthrocytes ($10^5$) from donor.

Day 69   and thereafter:  Thin blood smears taken daily or every two days as required until monkeys recovered.  Those not recovering were treated by intramuscular injection of chloroquine when parasitaemia exceeded 10%.

Serum samples were taken when indicated and analysed for their antibody specificity.  Each serum was screened by:

i)   indirect immunofluorescence against acetone-fixed smears of parasitized red cells,

ii)  radioimmunoassay (RIA),

iii) Western blotting against purified P.195 protein (and specific fragments), extracts of merozoites and extracts of total blood stage forms,

iv)  their ability to immunoprecipitate $^{35}$S methionine labelled protein from detergent extracts of parasitized cells.

c)   Results of the challenge.

In Group 1 there were detectable parasites in all animals at day 7 and the experiment was terminated by the administration of chloroquine to animals 1 and 2 on day 13 and animal 3 on day 18 after challenge, when the parasitaemia in each animal rose above 10%.

In Group 2 one animal, Number 5, successfully controlled the infection with a parasitaemia not rising above 1%, and from day 29 no parasites were detected in blood smears.  Animal Number 6 showed a course of infection no different from the control animals in Group 1 and was treated with chloroquine on day 13. The third animal (Number 4) appeared to be controlling a heavy infection, with a falling parasitaemia on days 19 and 20, but died on day 22.

In Group 3 two animals, Numbers 9 and 10 failed to control the infection and had a similar course of infection to Animals 1 and 3, respectively, in the control Group 1. The two remaining animals, Numbers 7 and 8 controlled their infections. A peak parasitaemia of 4% was seen in animal 7 on day 18 after challenge and the parasites could not be detected in blood smears after day 26. In animal 8 a peak parasitaemia of 2.3% on day 14 was cleared by day 22.

d)   Analysis of the antibody response of the immunised animals.

i)   Immunofluorescence

The antibody titre against acetone-fixed smears of P. falciparum-infected red cells was determined for sera from the animals prior to immunization and approximately 7 days after the primary, secondary and tertiary injections. In Group 1 no detectable specific antibodies were present at the lowest serum dilution (1/50) in any of the samples. In Group 2 specific antibodies were detected after primary or secondary immunization and the specific titre appeared to increase after the tertiary immunization to 1 in 800 (animal 6), 1 in 1600 (animal 4) and 1 in 3200 (animal 5). In Group 3 only animal 10 appeared to have parasite specific antibodies (at a titre of 1 in 100) after the immunization schedule.

ii) Radioimmunoassay

The serum samples from the animals after the third immunization were analysed by RIA for their response to purified P.195 in a microtitre plate assay. Sera from rabbits immunised with the purified P.195 protein and with the individual recombinant proteins were included as controls. Some antibody was detected

MJWD/AS/12th June 1987

(at 1 in 25 dilution) in the sera from all the animals in Groups
2 and 3.

When the response to the total trpE fusion protein or the
β-galactosidase carrier was examined it was clear that the
animals in Group 3 had responded extremely well to the E.
coli-derived part of the polypeptide.

iii) Western blotting

The antisera were analyzed by Western blotting using the
following antigens:

a)  purified P.195 protein and specific fragments with or without
    prior reduction with dithiothreitol
b)  total infected red cells
c)  total merozoites with or without prior reduction with
    dithiothreitol.

All the animals in Groups 2 and 3 produced antibodies that
recognised purified P.195 and the 150 kd fragment; the animals in
Group 2 also produced antibodies against the 110 and 83 kd
fragments.  In extracts of schizonts the antibodies from the
animals in Group 2 reacted with a large number of polypeptides,
in particular species of 70 to 80,000 kd.  These species were
also present in the merozoite extracts.  The P.195 and the 150 kd
species were recognised by the antibodies in Group 3 sera, and
additional species of about 45 and 42 kd were detected by the
sera from animals 9 and 10.  These lower molecular mass species
are derived from the C-terminus of the P.195.  In extracts of
merozoites there was little reaction of the Group 3 sera with any
of the antigens.

iv)  Immunoprecipitation

When the antisera were used to immunoprecipitate [35]S-methionine
labelled polypeptides from extracts of early intracellular
parasites ('rings') or asynchronous parasites, a large number of

MJWD/AS/12th June 1987

polypeptides were recognised by the animals in Group 2. In Group 3 only animal 10 appeared to produce antibodies specific for P.195 in this assay.

The protection achieved by immunization with the recombinant protein was as good as that achieved using the whole parasite as the immunogen. The animals immunized with whole merozoites produced antibodies against a spectrum of polypeptides, whereas the animals immunised with the recombinant proteins appeared to give a specific response to P.195. In the test group the response to the P.195 antigen was most clearly detected by Western blotting, a technique that is probably more sensitive for the detection of antibodies against 'denatured' antigen. Only animal 10 appeared to respond reasonably well when assayed by immunofluorescence or immunoprecipitation, but this animal was not better protected than other animals in this group.

Example 6:  Epitope analysis of the C-terminal 292 amino acid sequence of the P.195 gene

B-cells expressing surface IgM (and eventually soluble immunoglobulin molecules) recognise protein epitopes that are usually sterically exposed and often composed of 5 to 7 amino acids for which a conformational spatial arrangement is crucial. Algorithms to define hydrophilic regions of proteins (eg. Hopp and Woods, Proc. Natl. Acad. Sci. USA 78, (1981) 3824) have been used to predict such antigenic determinants. Epitopes recognized by T-cells are often linear and it has been suggested that they may form an amphipathic helix (DeLisi, and Berofsky, Proc. Acad. Sci. USA 82, (1985) 7048), allowing interaction with the Class II major histocompatibility proteins on the surface of antigen presenting cells and the specific receptor of an appropriate T-cell. The amino acid sequence of the P.195 component of the hybrid protein was examined by secondary structure predictive algorithms (eg. according to Chou and Fasman, Biochemistry 13, (1974) 222) to identify potential helical structures. Alpha-helix structure was predicted for residues 1350-1363, 1385-1402, 1417-1445, 1451-1476, 1492-1528, 1532-1540, 1548-1561, 1583-1591 and 1628-1640. When these regions were examined further using the hydrophilicity predictions of

MJWD/AS/12th June 1987

Hopp and Woods, regions 1350-1363, 1532-1540, 1548-1561 and 1583 to 1591 were moderately or highly hydrophilic, whereas the other regions (with the exception of 1628-1640 which is very hydrophobic) were predicted to be amphipathic helices, i.e. sequences predicted to be neither grossly hydrophilic or grossly hydrophobic and in which there is segregation of hydrophobic and hydrophilic residues to opposite sides of the helix (one turn of the helix represents 3.6 residues, i.e. $100^{\circ}$ twist per residue). Therefore, it can be predicted that potential helical segments 1350-1363, 1532-1540, 1548-1561 and 1583 to 1591 may be the whole or part of B-cell epitopes recognised by antibodies, whereas helical segments 1385-1402, 1417-1445, 1451-1476 and 1492-1528 constitute potential T-cell epitopes capable of being recognised by a T-cell with the appropriate receptor when presented in conjunction with a major histocompatibility Class II protein.

Example 7:   Expression of CSP/P.195 Fusion Proteins in E. coli

a)   type/CSP trpE P.195 hybrids.

About 2μg of DNA of the plasmids pfO$_1$trp75, pfO$_1$trp77 and pfO$_1$trp750 (A.J. Makoff; submitted for publication) were digested with the restriction enzymes BamH1 and Hind III using conditions suggested by the manufacturer, and then electrophoresed through a 0.8% agarose gel in a Tris-borate-EDTA buffer containing ethidium bromide. The DNA fragments were visualised by UV illumination and the regions containing the 4.3kb, 4.1kb and 4.28kb BamH1/Hind III fragment from the PfO$_1$trp75, pfO$_1$trp77 and pfO$_1$trp750 plasmids, respectively, were eluted and purified.

A DNA fragment containing the coding sequence for the C-terminal end of the P.195 protein was cut out of a plasmid containing the EcoR1-Hind III fragment from pPfc1028 (nucleotides 3555 to 5920 in Holder et al., Nature, 317 (1985), 270) by digesting this plasmid with Xho II and Hind III. Four micrograms of DNA was digested with 8 units of Xho II in 50μl Xho II buffer at 37&C for 3.25 hours. At this time 0.5μl 5M NaCl and 20 units Hind III were added and the digestion was continued for two hours. The digested DNA fragments were resolved by electrophoresis on a 0.8% agarose gel. A 1462bp fragment (nucleotides 4459 to 5920) that contains the coding sequence for the C-terminal 293 amino acids (from residue 1348) was eluted and purified.

Aliquots of the Xho II-Hind III fragment were mixed with the BamHl-Hind III fragment of the vectors and treated with T4 DNA ligase in a final volume of 10μl ligation buffer. The ligation mix was used to transform competent DHl cells. A 4 to 50 fold stimulation in the number of transformants compared to the vector DNA self ligation controls was observed. Transformants were analysed to ascertain that they contained the correct plasmids with the inserted DNA. A sample of approximately 1μg plasmid DNA from one clone of each was digested with BamHl at the unique BamHl site (recreated by the ligation of the BamHl sticky end with the Xho II sticky end) in the presence of 0.5μl (12 units) of calf intestinal phosphatase. After digestion for 2 hours at 37°C the linearized plasmid DNAs were electrophoresed through a 0.8% agarose gel and then eluted and purified.

pCSP600/3 was grown overnight in 10ml LB medium containing 100 μg/ml ampicillin. The plasmid DNA was prepared and then digested with EcoRl and BamHl (48 units of each enzyme) in 100μl BamHl digestion buffer for 2 hours at 37°C. The digested DNA was electrophoresed through a 0.8% agarose gel and the 583 bp fragment was eluted and purified. The purified fragment was further digested with Xho II (2.5 units) in a final volume of 20μl for 2 hours at 37°C. The digestion products were electrophoresed on a 2% agarose gel and two fragments, those of 348 bp and 192 bp were purified.

Aliquots of both the 348 bp and the 192 bp fragments were mixed with the BamHl linearized, phosphatase-treated vectors in a final volume of 10μl ligation buffer and 0.3μl T4 ligase at 4°C for 65 hours and 15°C for 2 hours. The ligated DNA was used to transform competent DHl cells. Individual clones were grown up in overnight cultures and the plasmid DNA in each was analysed by restriction enzyme digestion. Aliquots of the overnight cultures were also diluted 1 in 5 in LB medium containing 100μg/ml ampicillin and 10μg/ml indole acrylic acid to induce expression from the trp promotor. After 5 hours these cultures were harvested by centrifugation, and the cell pellets were dissolved in a buffer containing sodium dodecyl sulphate (SDS).

MJWD/AS/12th June 1987

Proteins present in the cell lysates were separated by electrophoresis in 10% polyacrylamide gels in the presence of SDS, and then the proteins were either stained with Coomassie blue or transferred to nitrocellulose. The transferred proteins were probed with antibodies specific for the CSP or P.195. A number of recombinants were obtained which contained plasmids expressing fusion proteins reacting with both antisera. These fusion proteins contained either the first 35, 116 or 131 amino acids of the trpE gene product (derived from either pfO$_1$trp77, pfO$_1$trp750 or pfO$_1$trp75, respectively) followed by a part of the CSP repeat region and then the C-terminal 293 amino acids of P.195 (starting at amino acid residue 1348). The predicted structure of these recombinant proteins is shown in Table 2. It can be seen that recombinants containing either 1,2 or 4 copies of the 192 bp Xho III fragment were obtained. The molecular mass of the fusion proteins estimated from the polyacrylamide gel electrophoresis is compared with the calculated molecular mass in Table 3.

E.coli strain 750/CSP(a)/P.195 has been deposited with the National Collection of Type Cultures and is registered as NCTC 11952.

b)    abbreviated trpE/CSP/P.195 hybrids.

The plasmid 750/CSP(a)/P.195 contains a single Tth III 1 site that is situated within the DNA coding for the trpE derived amino acid sequence, 440 bp from the Eco Rl site and 150 bp from the junction with the CSP repeat sequence. To produce fusion proteins containing less of this sequence the plasmid was opened at this site by restriction enzyme digested with Bal 31 and then religated. Expression of protein in the correct reading frame was determined by Western blotting of cell lysates.

Ten microgrammes of the plasmid 750/CSP(a)/P.195 was digested to completion with the enzyme Tth III 1. The linearized plasmid was treated with the exonuclease Bal 31 for 30, 60 and 120 seconds, repaired with DNA polymerase Klenow fragment and then recircularized with T4 ligase at 4°C overnight. The DNA was used to transform DH 1 cells and ampicillin resistant clones were selected on agar plates. Sixty individual strains were picked and the plasmids were analysed by

MJWD/AS/12th June 1987

restriction mapping with Eco R1 and Bam H1.  Plasmids of interest contained the Bam H1 fragment of 348 bp coding for the CSP repeat and a smaller Eco R1-Bam H1 fragment coding for the amino sequence terminal *trp*E sequence.  Strains containing these plasmids were analyzed further by western blotting of cell lysates.  In the strains 750/CSP(a) P.195 Tth III 1 Bal 31 6a, 4b, 11b and 12b the Eco R1-Bam H1 fragment was estimated at 235, 350, 330 and 320 bp respectively, compared with a 591 bp fragment in the parent 750/CSP(a)/P.195 plasmid.  In the strains 6a, 4b and 11b the Nru 1 site 140 bp 5' to the Tth III 1 site had been removed

c)  CSP/P.195 hybrids with minimal additional sequence.

The plasmid pCSP 600/3 was opened at the BamH1 site and ligated with a 1838bp XhoII fragment of 507 pPfc1028 EcoR1-Hind III.  This fragment contains the coding sequence for the terminal 292 amino acids of the P.195 protein followed by the stop codon and an additional 876 bp of untranslated sequence.  Expression of this construct (CSP 600/3/P.195) results in a single protein species driven by the *tac* promoter containing epitopes of both the CSP (46 repeats) and the P.195 protein.

The plasmid 750/CSP(a)/P.195 (20µg) was digested with 40 units of Sal 1 at 37°C for three hours.  After ethanol precipitation the DNA was redissolved in buffer and then digested with 36 units of Bam H1 enzyme at 37°C for 7 minutes.  The products were fractionated on a 0.8% agarose gel and the 2431 base pair partial digest fragment was eluted and purified by binding to an Elutip column.  The plasmid pXY461 (16µg) was digested with 40 units of Sal 1 at 37°C for 3 hours and then with 36 units of BamH1 at 37°C for 3 hours.  The products were electrophoresed through agarose and the 3253 bp fragment was eluted and purified.  The two DNA fragments were mixed and ligated with T4 · ligase and then used to transform TG-1 cells to ampicillin resistance. Transformants were plated on agar containing ampicillin and X-gal, and white colonies derived from strains in which the galactosidase gene in the plasmid had been lost, were picked and analysed.  Ten out of 12 strains picked contained plasmids with the correct construction, in which the *tac* promoter is followed by an open reading frame coding for

MJWD/AS/12th June 1987

the amino acid sequence MNSPSMG/DP(NANP)$_{26}$NKNNQGNGQG/DPYK...
(N.terminal sequence/CSP sequence/C-terminal sequence of P.195).  One
of these strains was chosen and named 461/CSP(a)/P.195.


Table 2   Predicted Structure of the Expression Products


Strain    trpE sequence/CSP sequence/C-terminal sequence of P.195

$\qquad\quad$ 35 $\qquad\qquad\qquad\qquad$ 1348

77/CSP(a)/P.195   ...RPAT/DP(NANP)$_{26}$NKNNQGNGQG/DPYK....

$\qquad\qquad\quad$ 116 $\qquad\qquad\qquad\qquad$ 1348

750/CSP(a)/P.195   ...DEDA/DP(NANP)$_{26}$NKNNQGNGQG/DPYK....

$\qquad\qquad\quad$ 131 $\qquad\qquad\qquad\qquad$ 1348

75/CSP(a)P.195   ....RLLQ/DP(NANP)$_{26}$NKNNQGNGQG/DPYK....

$\qquad\qquad\quad$ 35 $\qquad\qquad\qquad$ 1348

77/CSP(b)/P.195   ....RPAT/DP(NANP)$_{15}$NV/DPYK....

$\qquad\qquad\quad$ 116 $\qquad\qquad\quad$ 1348

750/CSP(b)/P.195 ....DEDA/DP(NANP)$_{15}$NV/DPYK....

$\qquad\qquad\quad$ 131 $\qquad\qquad\quad$ 1348

75/CSP(b)/P.195 . ...RLLQ/DP(NANP)$_{15}$NV/DPYK....

$\qquad\qquad\quad$ 116 $\qquad\qquad\qquad\qquad\quad$ 1348

750/CSP(b2)/P.195....DEDA/DP(NANP)$_{15}$NVDP(NANP)$_{15}$NV/DPYK....

$\qquad\qquad\quad$ 116

750/CSP(b4)/P.195   ..DEDA/DP(NANP)$_{15}$NVDP(NANP)$_{15}$NVDP(NANP)$_{15}$

$\qquad\qquad\qquad\qquad$ 1348
$\qquad\qquad$ NVDP(NANP)$_{15}$NV/DPYK....


CSP600/3/P.195     MNSHPNANPNVDPNANPNVDP(NANP)$_{15}$NVDP(NANP)$_{26}$
$\qquad\qquad\qquad\qquad$ 1348
$\qquad\qquad\qquad\qquad$ NKNNQGNGQG/DPYK....

$\qquad\qquad\qquad\qquad\qquad\qquad$ 1348
461/CSP(a)/P.195     MNSPSMG/DP(NANP)$_{26}$NKNNQGNGQG/DPYK.....


The sequences are presented in the standard single letter code for
amino acids.  The junctions between the different elements (/) are
indicated and the numbering indicates the amino acid residue in the
trpE and in the P.195 sequences.


MJWD/AS/12th June 1987

Table 3   Molecular masses of the fusion proteins

| Strain | Calculated mass | Apparent mass (SDS-PAGE) |
|---|---|---|
| 77/CSP(a)/P.195 | 49,276 | |
| 750/CSP(a)/P.195 | 57,752 | 62,000 |
| 75/CSP(a)/P.195 | 59,501 | 62,500 |
| 77/CSP(b)/P.195 | 44,122 | 47,000 |
| 750/CSP(b)/P.195 | 52,598 | 54,000 |
| 75/CSP(b)/P.195 | 54,347 | 54,000 |
| 750/CSP(b2)/P.195 | 58,963 | 66,000 |
| 750/CSP(b4)/P.195 | 71,693 | 93,000 |
| CSP600/3/P.195 | 53,735 | |
| 461/CSP(a)/P.195 | 46,096 | 49,500 |

Example 8:      Expression of CSP/P.195 by recombinant Vaccinia virus

a)    Construction of plasmid pPfc1028 PD/SV40

A Pst 1-Dra 1 fragment comprising nucleotides 5271 to 5338 in the P.195 nucleotide sequence (Holder et al., Nature, 1985) was cloned into the plasmid pUC 9 that had been digested with Pst 1 and Hind II. The blunt end ligation recreates a stop codon (TGA) in place of the · TAA stop codon that is part of the Dra 1 recognition site, and one of the strains was chosen and named pPfc1028PD-1.

DNA from pPfc1028PD-1 was digested with BamH1 at the unique site and then ligated with a Bam H1-Bgl II fragment derived from SV40 virus DNA and containing a termination sequence (Subramani, et al, Mol. Cell

MJWD/AS/12th June 1987

Biol 1:854 1981). The ligation products were used to transform DH1 cells. The plasmid DNA in six ampicillin resistant strains was analysed by restriction mapping using Pst 1 and Bam H1. Three of the six clones contained the SV40 termination sequence in the correct orientation, the remainder contained the sequence in the opposite direction. One of the correct constructions was chosed and named pPfc1028 PD/SV40.

b)   Expression of the CSP/P.195 hybrid by vaccinia virus.

DNA coding for the CSP/P.195 hybrid was inserted into a vaccinia virus vector under the control of the p11k promoter. In this construction the SV40 termination sequence was used in place of the natural P.195 3' nontranslated sequence. The transfer vector pVp11k contains a Cla 1-Eco R1 fragment from the vaccinia p11k promoter inserted into the Cla 1-Eco sites of vaccinia virus thymidine kinase gene in a pUC 9-based plasmid (Newton et al. in Vaccines 86. New approaches to immunization, eds Brown, F., Channock, R.M. & Lerner, R.A. pp 303-309; Newton et al. in Vaccines 87. Modern approaches to new vaccines, in press). DNA containing an open reading frame and inserted in the right orientation into the unique Eco R1 site will be transcribed under the control of the p11k promoter and translated into protein sequence in a recombinant vaccinia-infected cell.

A three fragment ligation was performed. Plasmid pVp11k (4 ug) was digested with Eco R1 (10 units) together with 12.5 units of calf intestinal phosphatase for 5 hours at 37°C. The linearized, dephosphorylated plasmid was electophoresed through an agarose gel, electroeluted and then purified. Plasmid 461/CSP(a)/P.195 (2.6 ug) was digested with Eco R1 and Pst 1 and the fragment containing the coding region for the CSP repeats together with the P.195 coding region to the Pst 1 site was eluted. DNA for the remainder of the P.195 coding sequence, together with the SV40 termination sequence was obtained by digestion of the plasmid pPfc 1028 PD/SV40 with Pst 1 and Eco R1. These three fragments were ligated with T4 ligase and the products were used to transform DH 1 cells. Forty two colonies were investigated by restriction mapping of plasmid DNA with Eco R1 + Pst 1, with Cla 1 and with Bam H1. Four of these strains contained all

MJWD/AS/12th June 1987

three fragments in the correct orientation. One of these, pVp11k/CSP(a)/P.195 was chosen for further analysis. The open reading frame is the same as that in 461/CSP(a)/P.195. Vaccinia recombinants were obtained using the methods described by Mackett et al. (J. Virology 49: (1984) 857). The purified plasmid DNA was used to transfect CV-1 cells infected with vaccinia virus. Selected viral plaques resistant to 5-bromodeoxyuridine were analysed and 6 out of 12 of these expressed a protein that reacted with specific antibodies (raised against the CSP peptide-BSA conjugate, P.195 and expressed products from PME2 and 750/CSP(a)/P.195) on western blots of cell lysates.

Example 9: Expression of CSP/P.195 fusion proteins by recombinant baculoviruses in insect cells.

The plasmid pAc 360 (Smith et al. Mol. Cell. Biol. 3: (1983) 2156) contains DNA coding for the first 11 amino acids of polyhedrin followed by a synthetic Bam H1 linker and then the polyhedrin sequence 3' of the natural Bam H1 site at +177 relative to the initiator ATG. It was digested to completion at this unique Bam H1 site and then ligated with a 1461 bp Xho II fragment from a derivative of pPfc 1028 in which the Hind III site has been converted to a Bgl II/Xho II site. This created the plasmid pAc 360 195c which contains the coding region for the amino acid sequence MPDYSYRPTIGP/DPYK ..... (Polyhedrin N-terminus/292 amino acids from P.195 C-terminus).

pAc 960 contains DNA coding for the first 11 amino acids of polyhedrin followed by a synthetic Bam H1 linker ligated close to the end of the polyhedrin open reading frame. This plasmid was digested with Bam H1 and then ligated with two Bam H1-Pst 1 fragments, one was the Pst 1-Bam H1 fragment derived from the plasmid pPfc 1028 PD-1 (Example 8) and the other was the Pst 1-partial Bam H1 1158 bp fragment from 750/CSP(a)/P.195 containing the CSP repeat coding sequence and from the Xho II site to the Pst 1 site in the P.195 gene. This generated a plasmid pAc CSP(a) P.195 3' in which the polyhedrin promoter is followed by the coding sequence for MPDYSYRPTIGP/DP(NANP)$_{26}$NKNNQGNGQGDPYK ... (polyhedrin/CSP repeat/P.195 C terminus).

MJWD/AS/12th June 1987

pAc 360 195c and pAc CSP(a) P.195Δ3' were digested with Eco R1 and Pst 1 and used to construct pAc CSP(a) 195c. In this construct the open reading frame following the polyhedrin promoter is identical to that in pAc CSP(a) P.195Δ3' and the 3' untranslated sequence from the P.195 gene is present.

These plasmids were used to generate recombinant baculoviruses using the methods described by Smith et al. (supra). Occlusion negative plagues were identified by visual screening and viruses were analysed by Southern blotting. Expressed proteins were detected by Coomassie blue staining after SDS-PAGE and by Western blotting with specific sera.

Example 10:    Synthesis of a peptide corresponding to part of the CSP repeat and its conjugation to a carrier protein.

A peptide was synthesised by the solid phase technique (Merrifield et. al., Ann. Rev. Biochem. 39 (1970), 841) with the sequence (S-acetamidomethyl)cysteine-(Asn-Ala-Asn-Pro)$_4$-Asn- Ala and coupled to succinylated bovine serum albumin using a water-soluble carbodiimide. 200 nmole (71.5 mg) succinylated bovine serum albumin was mixed with 10 μmoles (19.6 mg) peptide in 2ml 0.1M sodium phosphate, pH 4.75. Twenty μmoles (3.8 mg) of 1-ethyl-3-(3-dimethylaminopropyl) carbodiimide was added and the solution was left for 16 hours at 22°C. The reaction was stopped by the addition of 3ml 1M sodium acetate, extensive dialysis against 1% acetic acid and freeze-drying.

Example 11:    Purification of the Fusion produced in E. coli

The fusion proteins produced by strains 750/CSP(a)/P.195 (NCTC 11952), 750/CSP(b)/P.195 and 750/CSP(b2)/P.195 were partially purified as follows.

Relatively pure preparations of the individual fusion proteins were produced from cell lysates. A single colony from a strain was grown up overnight at 37&C in 100 ml M9 medium containing 50 μg/ml ampicillin. The following day the overnight culture was diluted with 400 ml M9 culture medium containing 50 μg/ml ampicillin and 10 μg/ml

indole acrylic acid and incubated for 5 hrs at 37$^{o}$C. The bacteria were harvested by centrifugation at 6000g for 10 minutes. The bacterial pellet was suspended in 10 ml 25mM Tris pH 8.0 containing 1mM EDTA, 1 mM PMSF, 0.2% (v/v) NP40 and 1mg/ml lysozyme and left on ice for 2 hrs. After this time 20$\mu$l of 1M MgSO$_4$ and 200$\mu$l of 1 mg/ml DNAse were added and the sample was left to incubate for a further 2 hrs on ice water. The insoluble material was harvested by centrifugation at 20,000g for 10 minutes and the supernatant (S1) was retained. The pelleted material was washed by suspension in 10 ml 50mM Tris-HCl pH 8.0 containing 5mM EGTA, 5mM EDTA, 1 mM PMSF and 1% NP40. The material was centrifuged at 20,000g for 10 minutes and the supernatant was retained (S2). The pellet was resuspended in 10ml 50mM Tris HCl pH 8.1, 5mM EDTA, 0.5M KSCN, and then centrifuged at 20,000g for 10 min to yield a third supernatant (S3) and a pellet fraction (P). The pellet fraction was resuspended in 5mlH$_2$O and then dialysed extensively against 0.89% NaCl. Aliquots of each supernatant and pellet fraction were analysed by SDS-PAGE and Coomassie blue staining. This procedure resulted in a final pellet fraction that contained predominantly the fusion protein and therefore constituted an effective purification of the fusion protein.

Further purification of the 750/CSP(a)/P.195 fusion protein by chromatography was carried out. The final pellet fraction was suspended in 50mM Tris-HCl pH 8.5, 8M urea, 1 mM EDTA, 50mM NaCl, 100 mM dithiothreitol (DTT) and insoluble material was removed by centrifugation. The protein was further purified by gel filtration in this buffer (containing 1 mM DTT instead of 100mM) on a column of Superose 6 (Pharmacia). Alternatively the solution was adjusted to 1M ammonium sulphate and applied to a column of Phenyl-Sepharose equilibrated with this buffer. Under these conditions the fusion protein bound to the column and could be eluted by decreasing the ammonium sulphate concentration.

Example 12:    Immunogenicity of the hybrid proteins

The partially purified proteins were used to immunise rabbits by intramuscular inoculation with 0.5mg protein in Freund's Complete Adjuvant, with boosting doses on days 21 and 35 with 0.5mg protein in

MJWD/AS/12th June 1987

Freund's Incomplete Adjuvant. On day 56 after the primary immunisation serum samples were collected. Further boosts were given on days 98 and 125 and serum samples were collected on day 132.

a) Antibody response to the CSP repeat and the P.195 sequences assessed by radio-immunoassay.

Two substrates were used to assay the antibody response in animals immunised with the fusion proteins. The first substrate was P.195 purified from extracts of P. falciparum (Holder and Freeman, 1984, supra). The second was a synthetic peptide containing the CSP repeat conjugated to a carrier protein. The proteins were used to label wells of a microtitre plate for the radioimmunoassay (RIA) as described: European patent application no. 8504429.

The results in Table 4 show that the fusion proteins are immunogenic and elicit antibodies against both P.195 and the CSP amino acid sequence.

b) antibody response to the P.195 protein assessed by, immunoprecipitation, western blotting and immunofluorescence.

P. falciparum schizonts were biosynthetically labelled with $^{35}$S-methionine and then a cell-free extract was prepared (Holder and Freeman, 1982 supra). Antibodies raised against the partially purified fusion proteins from 750/CSP(a)/P.195 recognised the intact P.195 in this extract of schizonts as demonstrated by immunprecipitation, SDS-PAGE and fluorography. From extracts of surface labelled merozoites the same serum immunoprecipitated the 42 and 19 kd fragments of the P.195 protein.

In an alternative approach P.195 was purified by monoclonal antibody affinity chromatography (Holder and Freeman, 1984 supra), electrophoresed through a polyacrylamide gel and then transferred to nitrocellulose. Western blotting with antisera raised against the 750/CSP(a)/P.195, 750/CSP(b)/P.195 and 750/CSP(b$_2$)/P.195 revealed a positive reaction of antibodies in these sera with the purified P.195

MJWD/AS/12th June 1987

protein.  All the three antisera reacted by immunofluorescence with blood stage parasites, on air-dried acetone fixed smears.

Table 4      Antibody Titration in the Sera of Immunised Animals

$I^{125}$-Protein A bound (cpm) and as a percentage of maximal counts bound in brackets, after 2 boost injections or 4 boost injections top and bottom figures, respectively)

Antiserum from
animal immunised    Serum dilution
with:               1:10        1:100        1:1,000      1:10,000  1:100,000

A. Titrated against P.195 protein

| | 1:10 | 1:100 | 1:1,000 | 1:10,000 | 1:100,000 |
|---|---|---|---|---|---|
| 750/CSPa/P.195 | 2287(45.1) | 1732(34.1) | 597(11.7) | 324(6.4) | 236(4.6) |
| Fusion protein | 2936(72.8) | 2251(55.8) | 632(15.7) | 259(6.4) | 142(3.5) |
| 750/CSPb/P.195 | 625(12.3) | 262(5.2) | 146(2.9) | 191(3.8) | 159(3.1) |
| Fusion protein | 3506(86.9) | 1711(65.6) | 575(14.2) | 189(4.7) | 142(2.8) |
| 750/CSPb$_2$/P.195 | 1151(23.5) | 336(6.6) | 185(3.6) | 166(3.3) | 197(3.9) |
| | 2498(61.9) | 1092(27.1) | 294(7.3) | 139(3.4) | 109(2.7) |
| P.195 | 5074(100) | 4977(98) | 3743(78) | 1791(35) | 793(16) |
| | 4033(100) | 3818(95) | 3145(78) | 1855(46) | 1011(25) |
| Normal Control | 620(12.2) | 264(5.2) | 217(4.2) | 230(4.5) | 222(4.4) |
| | 587(14.5) | 212(5.2) | 154(3.8) | 106(2.6) | 134(3.3) |

MJWD/AS/12th June 1987

B. Titrated aginst CSP peptide-BSA conjugate

| | | | | | |
|---|---|---|---|---|---|
| 750/CSPa/P.195<br>Fusion protein | 3365 | 1379 | 330 | 168 | 362 |
| 750/CSPb/P.195<br>Fusion protein | 2824 | 1674 | 729 | 598 | 1351 |
| 750/CSPb$_2$/P.195<br>Fusion protein | 3068 | 2588 | 382 | 416 | 271 |
| CSP peptide-BSA | 2947 | 3684 | 3312 | 2161 | 940 |
| Normal Control | 662 | 231 | 147 | 409 | 143 |

At the end of Example 7, section a) supra, reference is made to the deposition of E. coli strain 750/CSP(a)/P.195. Complete details of this deposit, which was made in accordance with the Budapest Treaty, are as follows.

(A)    NAME AND ADDRESS OF THE INTERNATIONAL DEPOSITARY AUTHORITY:

NATIONAL COLLECTION OF TYPE CULTURES
CENTRAL PUBLIC HEALTH LABORATORY
COLINDALE AVENUE
LONDON NW9 5HT
UNITED KINGDOM

(B)    DATE OF DEPOSIT:    9 APRIL 1986

(C)    ACCESSION NO.:    NCTC 11952

MJWD/AS/12th June 1987

1. A recombinant, conjugate protein comprising at least one epitope of each of the CSP and a blood stage antigen of a _Plasmodium_ parasite.

2. A recombinant, conjugate protein as claimed in claim 1 wherein the _Plasmodium_ species is _P. falciparum_.

3. A recombinant, conjugate protein as claimed in either claim 1 or 2, wherein the blood stage antigen component is P.195.

4. A recombinant, conjugate protein as claimed in any one of claims 1 to 3 wherein the shortest sequence containing a CSP epitope is selected from the group, A(n+1), A(n)B, BAB, ABA, BA$_{(n)}$ wherein A is Asn-Ala-Asn-Pro, B is Asn-Val-Asp-Pro, and n is an integer $\geq 2$.

5. A DNA sequence encoding for a recombinant, conjugate protein as claimed in any one of claims 1 to 4.

6. A vector containing a DNA sequence of claim 5.

7. A non-pathogenic virus provided with a DNA sequence as claimed in claim 5.

8. An expression vector containing a DNA sequence as claimed in claim 5, tandemly linked to a coding portion of a gene translatable by a lost and optionally, a control sequence(s) associated therewith.

9. A fusion protein comprising a host peptide, and a recombinant, conjugate protein as claimed in claims 1 to 4.

10. A vaccine comprising a recombinant, conjugate protein as claimed in any one of claims 1 to 4.

11. A vaccine as claimed in claim 10 additionally comprising an adjuvant.

MJWD/AS/12th June 1987

12. A vaccine as claimed in claim 11 wherein the adjuvant is aluminium hydroxide.

1.  A method of producing a recombinant, conjugate protein comprising at least one epitope of each of the CSP and a blood stage antigen of a _Plasmodium_ parasite said method comprising inserting a DNA sequence coding for said protein into a vector and transforming a host with said vector such that said protein is expressed by said host.

2.  A method of producing a recombinant, conjugate protein as claimed in claim 1 wherein said DNA sequence is obtained by
    a)  creating a cDNA or genomic DNA library from a _Plasmodium_ parasite,
    b)  selecting probes for the CSP and blood stage antigen DNA and rendering the said probes radioactive,
    c)  selecting a member or members of said library by use of said probes, and
    d)  isolating said DNA from said members.

3.  A method as claimed in claims 1 or 2 wherein the blood stage antigen is P.195.

4.  A method as claimed in any one of claims 1 to 3 wherein the shorter sequence containing a CSP epitope is selected from the group, A(n+1), A(n)B, BAB, ABA, BA(n) wherein A is Asn-Ala-Asn-Pro, B is Asn-Val-Asp-Pro and n is an integer $\geqslant 2$.

5.  A method as claimed in any proceeding claim, wherein expression is obtained in a bacterial, mammalian, or insect cell system.

6.  A method of manufacturing a vaccine comprising admixing a recombinant, conjugate protein comprising at least one epitope of each of the CSP and a blood stage antigen of a _Plasmodium_ parasite and a pharmaceutically acceptable carrier.

0250261

ATG AAT TCC CGG GGA TCC C
     ⎣___EcoR1___⎦    ⎣__BamH1__⎦
              ⎣____Sma1____⎦

Ptac    lacᶻ

pXY 460

Met  Asn  Ser  Pro  Ser  Met  Gly  Asp  Pro
ATG  AAT  TCC  CCA  TCG  ATG  GGG  GAT  CCC
      ⎣__EcoR1__⎦    ⎣___Cla1___⎦    ⎣___BamH1___⎦

Ptac    lacᶻ

pXY 461

# Fig. 1.

Fig.2.

Fig. 3.

Fig. 4.

0250261

pPfc1028 5271→5338
Pst1    Dra1

puc9

Eco R
Pst1  Hind II  Bam H1

Pst1 + Hind II

pPfc 1028 PD-1

Pst1    Bam H1

Bam H1

SV40 termination sequence
BglII                        Bam H1

1028 P D/SV40

Eco R1
Bam H1

Pst1
Bam H1 / BglII = XhoII

Fig. 5.

Fig. 6.

Fig. 7

Fig. 8.

0250261

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PROCEEDINGS OF THE NATIONAL ACADEMY OF SCIENCES OF THE USA, vol. 82, August 1985, pages 5121-5125; R.L. COPPEL et al.: "A blood stage antigen of Plasmodium falciparum shares determinants with the sporozoite coat protein" * Whole article * | 1-6 | C 12 N 15/00 C 12 N 7/00 A 61 K 39/015 C 12 P 21/00 |
| | --- | | |
| X | NUCLEIC ACIDS RESEARCH, vol. 13, no. 2, 25th January 1985, pages 369-379, IRL Press Ltd, Oxford, GB; I.A. HOPE et al.: "The gene for an exported antigen of the malaria parasite Plasmodium falciparum cloned and expressed in escherichia coli" * Whole article * | 1-6 | |
| | --- | | **TECHNICAL FIELDS SEARCHED (Int. Cl.4)** |
| X | NUCLEIC ACIDS RESEARCH, vol. 14, no. 7, April 1986, pages 3089-3102, IRL Press Ltd, Oxford, GB; H.D. STAHL et al.: "An asparagine-rich protein from blood stages of Plasmodium falciparum shares determinants with sporozoites" * Abstract * | 1,2,9 | C 12 N A 61 K |
| | --- -/- | | |

The present search report has been drawn up for all claims

| Place of search | Date of completion of the search | Examiner |
|---|---|---|
| THE HAGUE | 16-09-1987 | CUPIDO M. |

0250261

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| Y,D | SCIENCE, vol. 228, 24th May 1985, pages 958-962; J.F. YOUNG et al.: "Expression of Plasmodium falciparum circumsporozoite proteins in escherichia coli for potential use in a human malaria vaccine" * Whole article * | 1-6,9- 18 | |
| | --- | | |
| Y,D | NATURE, vol. 317, 19th September 1985, pages 270-273; A.A. HOLDER et al.: "Primary structure of the precursor to the three major surface antigens of Plasmodium falciparum merozoites" * Whole article * | 1-6,9- 18 | |
| | ----- | | TECHNICAL FIELDS SEARCHED (Int.Cl.4) |

The present search report has been drawn up for all claims

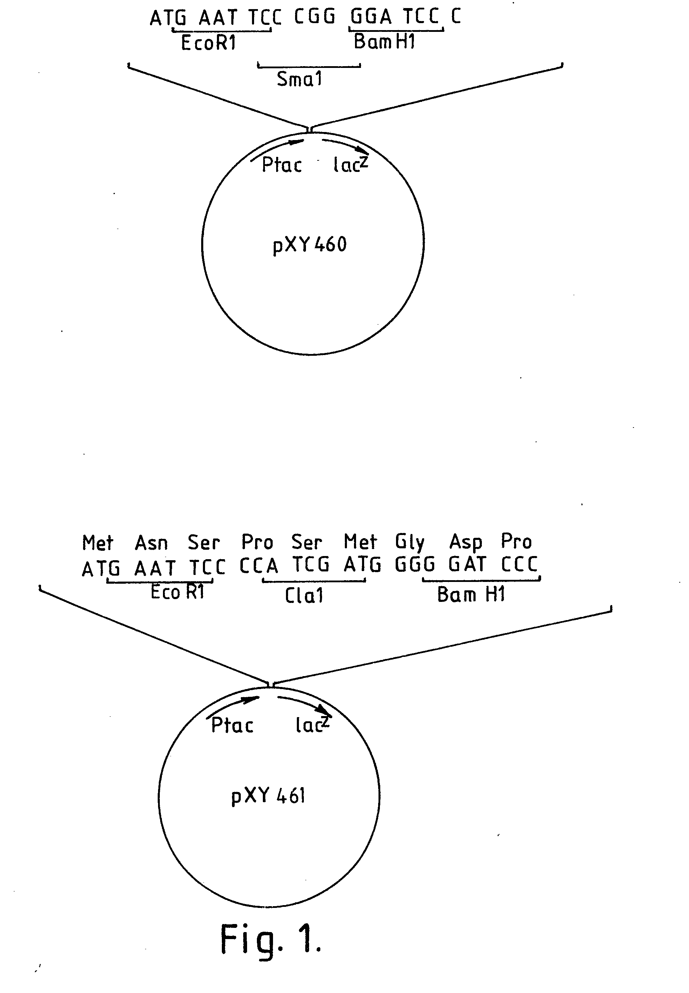

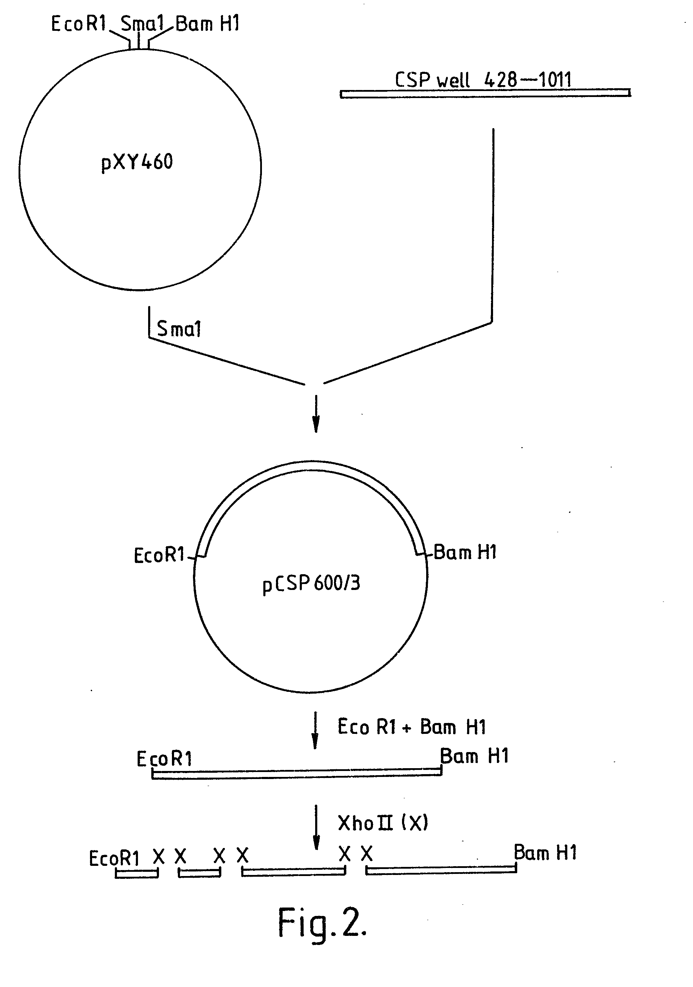

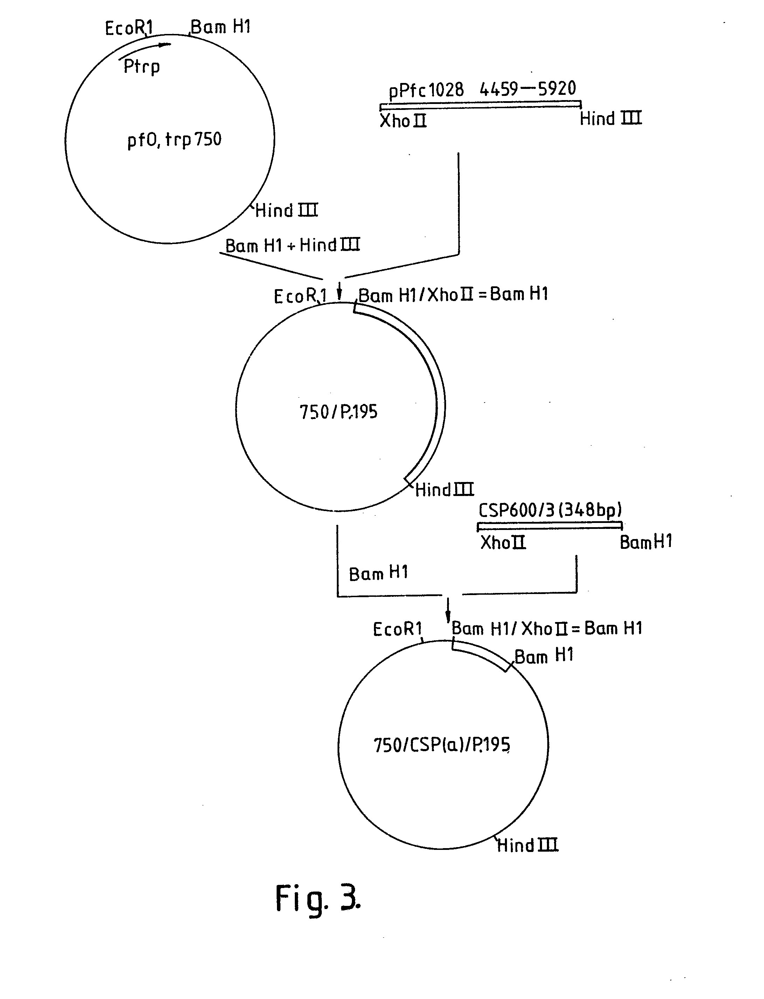

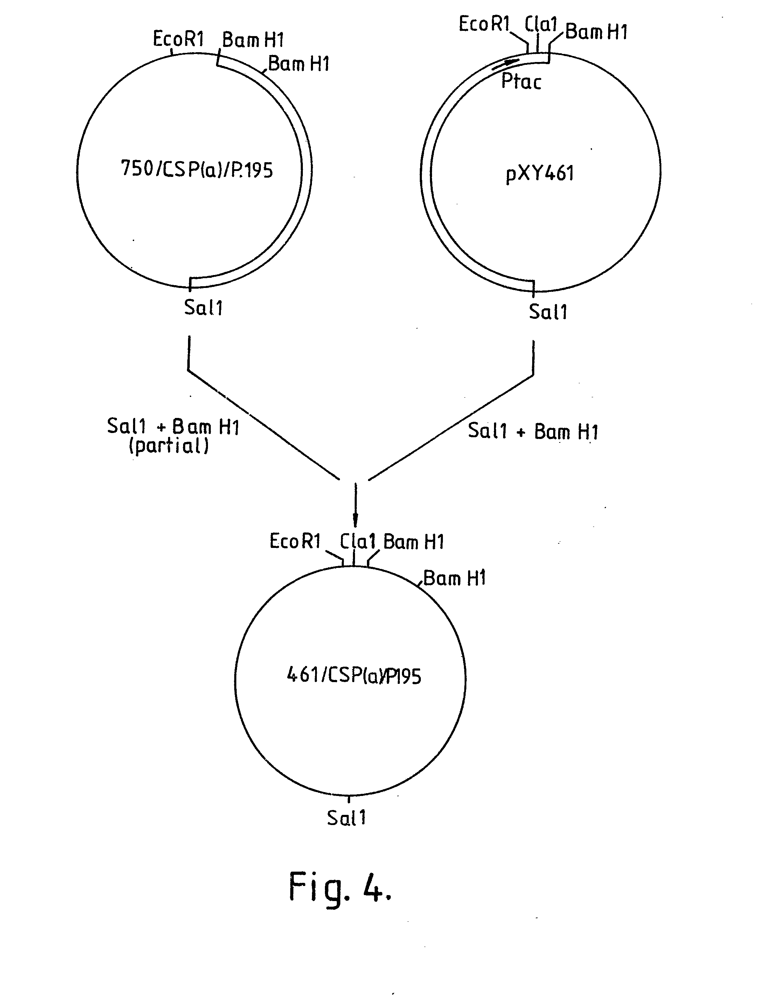

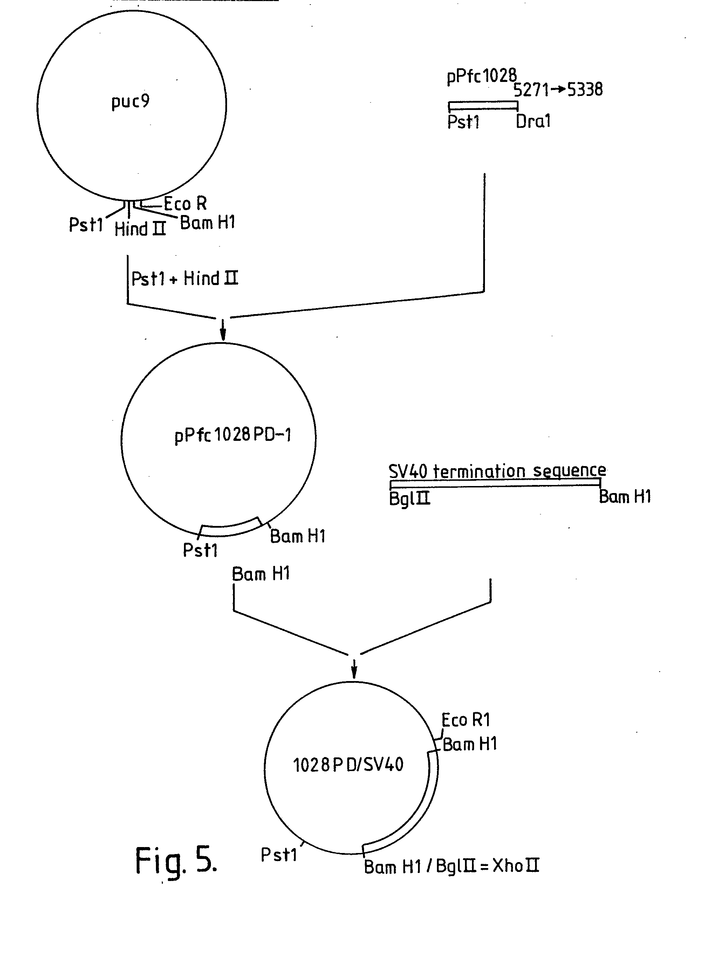

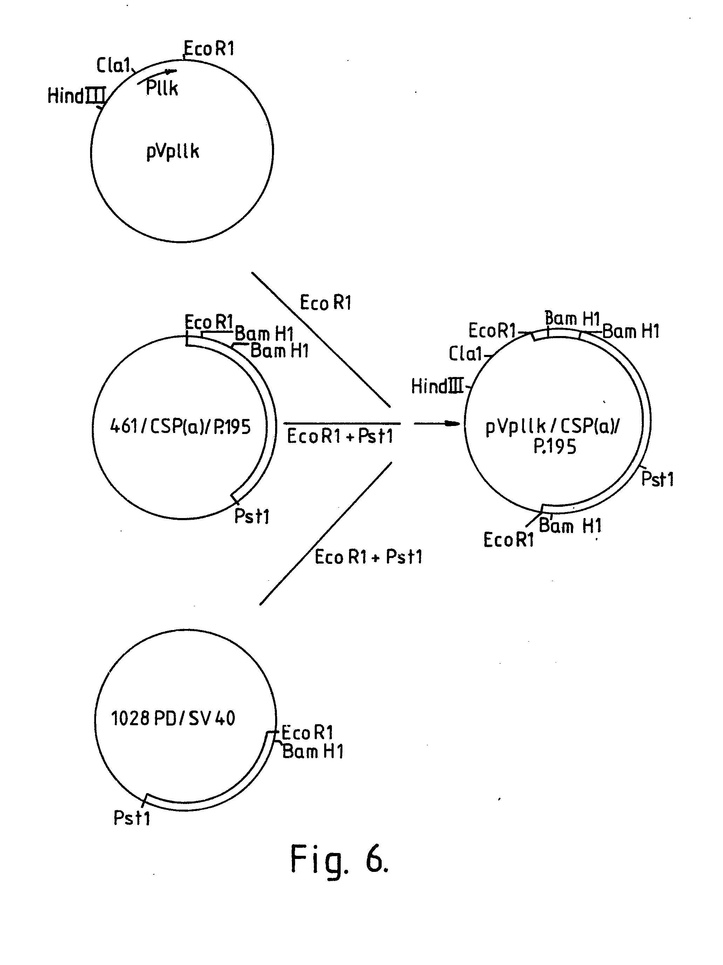

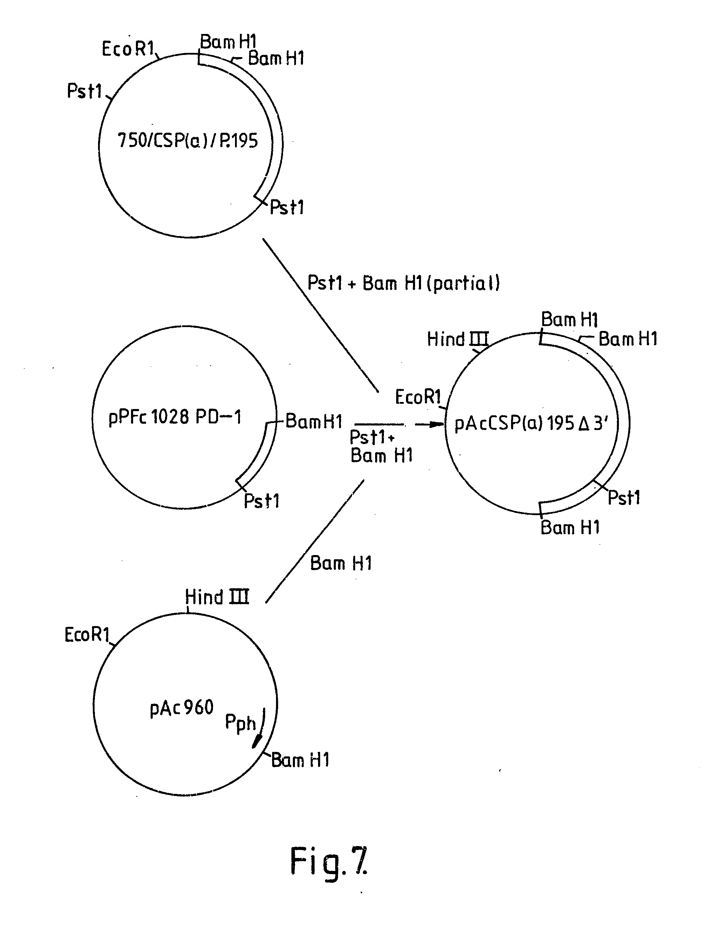

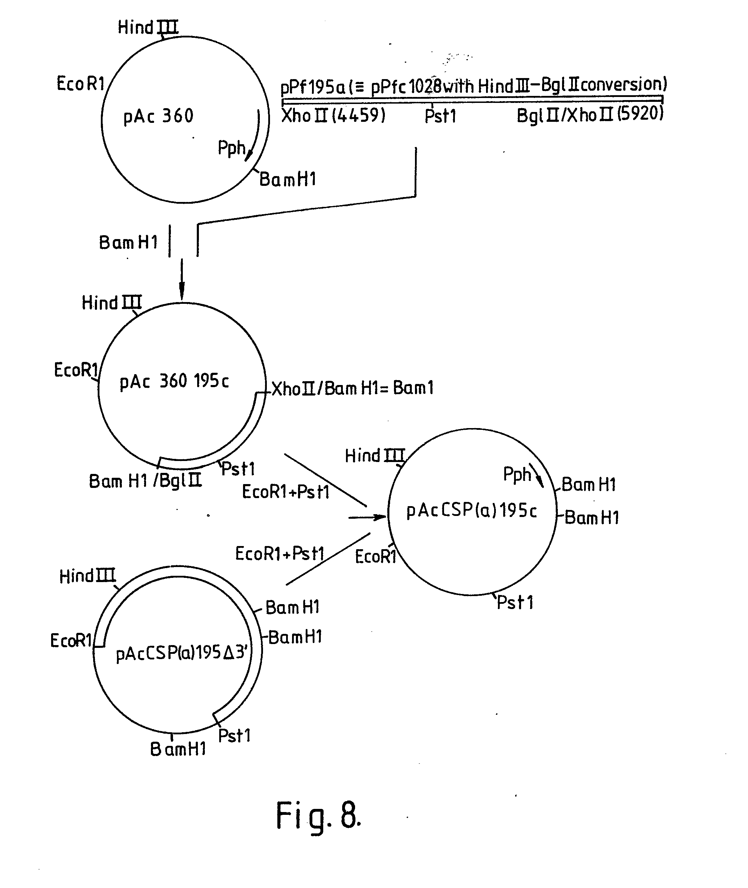

| Place of search | Date of completion of the search | Examiner |
|---|---|---|
| THE HAGUE | 16-09-1987 | CUPIDO M. |